# EUROPEAN PATENT APPLICATION

(11) **EP 4 446 328 A1**
(43) Date of publication of application: **16.10.2024**
(21) Application number: 22894671.1
(22) Date of filing: 08.11.2022
(51) Int. Cl.: C07F 5/02, A61K 41/00, A61K 51/00, A61P 35/00

(54) **NOVEL BORONSOME FACILITATING DIAGNOSIS AND TREATMENT**

(30) Priority: 22.11.2021 CN 202111391620
(71) Applicant: Peking University, Beijing 100871 (CN)
(72) Inventor: LIU, Zhibo, Beijing 100871 (CN); LI, Jiyuan, Beijing 100871 (CN); GUO, Zhibin, Beijing 100871 (CN)
(74) Representative: Cabinet Becker et Associés
(86) International application number: PCT/CN2022/130606
(87) International publication number: WO 2023/088134

(57) **Abstract**

The present disclosure provides a carborane-phospholipid conjugate, in which carboranyl is introduced at the end of the arm of a phospholipid. The present disclosure also provides a liposome composition comprising the carborane-phospholipid conjugate, and a use of the composition in delivering at least one therapeutic agent and/or at least one diagnostic agent.

## Description

The present application claims priority of the Chinese Patent Application No. 202111391620.9, filed on November 22, 2021, the disclosure of which is incorporate d herein by reference in its entirety as part of the present application.

### TECHNICAL FIELD

The disclosure relates to the field of medicine and diagnosis, in particular to a novel boronsome facilitating diagnosis and treatment.

### BACKGROUND

Boron delivery agents for boron neutron capture therapy (BNCT) are reviewed in Li J, Tu Z, and Liu Z, Brief Review of Development History of Boron Delivery Agents, Scientia Sinica Chimica, vol. 50, pp. 1296-1319, 2020. BNCT is a binary, cell-scale, highly targeted, and high linear energy transfer radiotherapy, which has excellent killing effect on locally aggressive malignant tumors, such as, melanoma, glioma, and recurrent head and neck cancer. The killing effect of BNCT on cells originates from alpha particles and recoil ⁷Li nuclei generated by nuclear fission reaction of non-radioactive ¹⁰B isotope capturing low-energy thermal neutrons, which have high linear energy transfer and high relative bioavailability, can cause lethal damage to cells, and have limited penetrability (5-9 µm, *i.e.*, approximately the diameter of a cell) so that the damage can be limited at single-cell scale. If a large number of ¹⁰B atoms can selectively accumulate in tumor cells and absorb sufficient neutrons to maintain the lethal ¹⁰B(n,α)⁷Li capture reaction, the BNCT will produce significant killing effect on malignant tumors and little effect on nearby normal tissue. The key to effective therapy for malignant tumors is to deliver a sufficient amount of boron to the site of tumor for performing a neutron irradiation.

At present, a widely used boron delivery agent is 4-dihydroxyboryl-L-phenylalanine (BPA). However, it has shortcomings such as insufficient boron content, which limits its efficacy. Use of uncommon boranes sometimes causes biological toxicity and immunogenicity. At the same time, the encapsulation and delivery of drugs by liposomes face a problem that the encapsulation structure is easily to be damaged. In the process of actual treatment, a physician further needs to diagnose the patient in time. However, the existing BNCT drugs are limited in their functions and can often only meet the requirement of treatment, which limits their clinical application. In this context, it is desired to find new materials to synthesize a molecule with novel structure to solve the above problems.

### SUMMARY

In an aspect, the present disclosure provides a carborane-phospholipid conjugate of Formula (I), 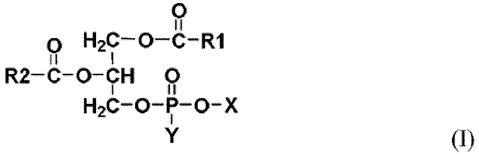 wherein: one of R¹ and R² is -L-carboranyl, and the other is a linear alkyl or alkenyl with 10-24 carbon atoms, wherein L is a divalent linking group; X is -H, - CH₂CH₂NH₃, -CH₂CH₂N⁺(CH₃)₃, -CH₂CH(NH₂)COOH, -CH₂CH(OH)CH₂OH, or 2,3,4,5,6-pentahydroxy-cyclohexan-1-yl; and Y is OH or O⁻.

In an embodiment of the present disclosure, the carboranyl in the -L-carboranyl is ¹⁰B-enriched.

In an embodiment of the present disclosure, X is -CH₂CH₂N⁺(CH₃)₃; and Y is O⁻.

In a preferred embodiment of the present disclosure, two lipid arms each comprising R1 or R2 satisfy one or more of conditions of: a difference in length within ± 1A; a distance between the lipid arms within 5A; and a dihedral angle within 5 degrees. In a more preferred embodiment of the present disclosure, the two lipid arms each comprising R1 or R2 satisfy any two of the three conditions. In an even more preferred embodiment of the present disclosure, the two lipid arms each comprising R1 or R2 satisfy all the three conditions.

In an embodiment of the present disclosure, the carboranyl is 1,2-C₂B₄H₅-, 1,2-C₂B₈H₉-, 1,2-C₂B₁₀H₁₁-, 2,3-C₂B₄H₇-, 7,8-C₂B₉H₁₂-, or 5,6-C₂B₈H₁₁-, preferably 1,2-C₂B₁₀H₁₁-.

In an embodiment of the present disclosure, L is -(CH₂)ₙ₊ₘ-, -(CH₂)ₙ₊ₘ-S-, -(CH₂)ₙ₊ₘ-O-, -(CH₂)ₙ-CH=CH-(CH₂)ₘ-, -(CH₂)ₙ-CH=CH-(CH₂)ₘ-S-, -(CH₂)ₙ-CH=CH-(CH₂)ₘ-O-, -(CH₂)ₙ-S-(CH₂)ₘ-, -(CH₂)ₙ-S-(CH₂)ₘ₊₁-S-, -(CH₂)ₙ-O-(CH₂)ₘ-, -(CH₂)ₙ-O-(CH₂)ₘ₊₁-O-, -(CH₂)ₙ-N(CH₃)-(CH₂)ₘ-, or -(CH₂)ₙ-N(CH₃)-(CH₂)ₘ₊₁-N(CH₃)-, wherein n is an integer from 1 to 20, and m is an integer from 0 to 20.

For example, the carborane-phospholipid conjugate of Formula (I) has a structure of: 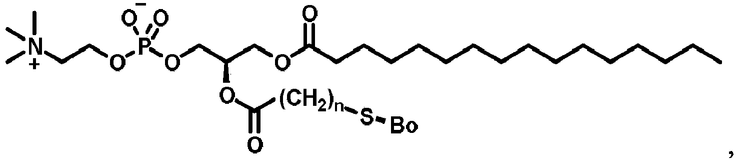 wherein Bo represents carboranyl, and n is an integer from 1 to 20. In an embodiment, Bo is 1,2-C₂B₁₀H₁₁-, preferably dicarba-closo-dodecylboranyl, more preferably 1,2-dicarba-closo-dodecylboranyl; and n is 3, 7, 11 or 15, preferably 11.

In another aspect, the present disclosure provides a liposome composition comprising the carborane-phospholipid conjugate, wherein the conjugate is a part of a lipid bilayer.

In an embodiment of the present disclosure, the liposome composition further comprises cholesterol.

In an embodiment of the present disclosure, the liposome composition further comprises a phospholipid, preferably dilauroylphosphatidylcholine (DLPC), dimyristoylphosphatidylcholine (DMPC), distearoylphosphatidylcholine (DSPC), dioleoylphosphatidylcholine (DOPC), dipalmitoylphosphatidylcholine (DPPC), dimyristoylphosphatidylglycerol (DMPG), distearoylphosphatidylglycerol (DSPG), dioleoylphosphatidylglycerol (DOPG), dipalmitoylphosphatidylglycerol (DPPG), dimyristoylphosphatidylserine (DMPS), distearoylphosphatidylserine (DSPS), dioleoylphosphatidylserine (DOPS), dipalmitoylphosphatidylserine (DPPS), dioleoylphosphatidylethanolamine (DOPE), dipalmitoylphosphatidylethanolamine (DPPE), dimyri stoylphosphatidylethanolamine (DMPE), distearoylphosphatidylethanolamine (DSPE), or dielaidoylphosphoethanolamine, more preferably DPPC.

In an embodiment of the present disclosure, the liposome composition further comprises a PEGylated phospholipid, e.g., DLPE-PEG, DPPE-PEG, DMPE-PEG, or DSPE-PEG, preferably DSPE-PEG.

In an embodiment of the present disclosure, the liposome composition further comprises at least one therapeutic agent and/or at least one diagnostic agent.

In a preferred embodiment, the liposome composition comprises at least one therapeutic agent, which is an anti-cancer drug. For example, the anti-cancer drug is selected from the group consisting of monomethyl auristatin E, monomethyl auristatin F, ibrutinib, acalabrutinib, zanubrutinib, doxorubicin, mitomycin-C, Mitomycin-A, daunorubicin, aminopterin, actinomycin, bleomycin, 9-aminocamptothecin, N8-acetylspermidine, 1-(2-chloroethyl)-1,2-dimethanesulfonylhydrazide, Yunnanmycin, gemcitabine, cytarabine, dolastatin, dacarbazine, 5-fluorouracil; paclitaxel, docetaxel, gemcitabine, cytarabine, 6-mercaptopurine, vincristine, cisplatin, oxaliplatin, and PARP inhibitors. In an embodiment, the anti-cancer drug is selected from the group consisting of PARP inhibitors, preferably olaparib, niraparib, rucaparib, fluzoparib, pamiparib, veliparib, talazoparib, more preferably olaparib.

In a preferred embodiment of the present disclosure, the liposome composition comprises at least one diagnostic agent, which is ⁶⁴Cu-NOTA-PEG2000-DSPE.

In still another aspect, the present disclosure provides use of the liposome composition for medicine.

In yet another aspect, the present disclosure provides use of the liposome composition for delivering at least one therapeutic agent and/or at least one diagnostic agent.

### BRIEF DESCRIPTION OF THE DRAWINGS

To illustrate the technical solutions of the embodiments of the present disclosure more clearly, the accompanying drawings of the embodiments are briefly introduced below. Clearly, the accompanying drawings described below only involve some embodiments of the present disclosure, but are not intended to limit the present invention.
FIG. 1: Schematic structural diagram of the novel boronsome and the encapsulation strategy of drug.
FIG. 2: Changes in tumor volume of mice under conditions of boronsome encapsulating different drugs (Dox: doxorubicin; PARPi: olaparib, a Poly(ADP-Ribose) Polymerase (PARP) inhibitor; and N represents neutron irradiation).
FIG. 3: Image of boronsome by transmission electron microscope (TEM).
FIG. 4: Full-body maximum intensity projection PET image of 4T1 tumor-bearing mice 12 h after intravenous injection of ⁶⁴Cu-NOTA-boronsome, wherein the tumors are outlined with white dashed lines.
FIG. 5: Cross-section of BoP-1 membrane structure simulated by molecular dynamics.
FIG. 6: Cross-section of BoP-2 membrane structure simulated by molecular dynamics.
FIG. 7: Cross-section of BoP-3 membrane structure simulated by molecular dynamics.
FIG. 8: Cross-section of BoP-4 membrane structure simulated by molecular dynamics.
FIG. 9: Cross-section of DPPC membrane structure simulated by molecular dynamics.
FIG. 10: UV-vis absorption of BoPs and DPPC measured under the condition that SRB is encapsulated.
FIG. 11: Leakage percentage of SRB measured after cultured in 50% bovine serum protein at 37°C for 24h.
FIG. 12: Boron uptake of 4T1 cells incubated with different concentrations of boronsome for 24h.
FIG. 13: Cell viability of 4T1 cells incubated with different concentrations of boronsome for 24h.
FIG. 14: Mean tumor volume of mice in the PARP inhibitor-boronsome and PARP inhibitor-boronsome + N treatment groups (n=9) (paired *t*-test, **p<0.01).
FIG. 15: Mean weight of each group of mice treated with boronsome + N or PARP inhibitor-boronsome + N (n=9).

### DETAILED DESCRIPTION OF THE INVENTION

To make the objects, technical solutions and advantages of the embodiments of the present disclosure clearer, the technical solutions of the embodiments of the present disclosure will be clearly and fully described below by reference to the accompany drawings of the embodiments of the present disclosure. It is obvious that the described embodiments are just some, but not all of the embodiments of the present disclosure. Based on the described embodiments of the present disclosure, all other embodiments obtained by those of ordinary skill in the art without creative effort will fall within the scope of protection of the present invention.

The present invention can be implemented in other specific forms without departing from the spirit of the present invention. It should be understood that any and all embodiments of the present invention can be combined with the technical feature(s) of any other embodiment(s) to obtain alternative embodiments on the premise of no conflict. The invention encompasses such combination to obtain alternative embodiments.

The technical or scientific terms used in present disclosure should have general meaning that will be understood by a person of ordinary skill in the art to which present invention belongs, unless otherwise defined.

As used in the present disclosure, the words 'including,' 'containing,' or 'comprising' or the like are intended to mean that an element occurred before the word encompasses an element listed after the word, as well as their equivalents, without excluding other elements.

All the numbers stated in the specification and claims expressing amounts of material, reaction conditions, durations, and quantitative properties of the material, etc., should be understood to be modified by the term 'about' in all cases, unless in the working examples or otherwise specified. It should also be understood that any numerical range listed in present application is intended to encompass all the subranges within the range and any combination of various endpoints of the range or subrange. For example, an integer from 1 to 20 comprises 1, 2, 3, 4, 5, 6, 7, 8, 9, 10, 11, 12, 13, 14, 15, 16, 17, 18, 19 and 20,and further comprises subranges of 1-3, 1-4, 1-10, 2-4, and 2-10, etc.

The present disclosure should be interpreted to be consistent with the rule and principles of chemical bonding. In some cases, hydrogen atom(s) can be removed to accommodate a substituent at a given position.

In present disclosure, the phospholipid refers to glycerophospholipids, that is, derivatives of compounds such as fatty acids (*e.g.*, capric acid, lauric acid, myristic acid, palmitic acid, stearic acid, oleic acid, or erucic acid), glycerol, and phosphoric acid, in which two hydroxyls of glycerol are substituted with fatty acid and the other hydroxyl is substituted with phosphoric acid or other groups, and a hydrophilic phosphate group is attached to tails of two hydrophobic lipid arms via glycerol molecule. The phospholipids mentioned in the present disclosure can be natural or synthetic phospholipids. Exemplary phospholipids comprise, but are not limited to, phosphatidic acid, phosphatidylcholine, phosphatidylethanolamine, phosphatidylserine, phosphatidylglycerol, and phosphatidylinositol.

In the present application, the carborane-phospholipid conjugate refers to phospholipid derivatives in which carboranyl is introduced at an end of lipid arm of the phospholipid.

In an aspect, the present disclosure provides a carborane-phospholipid conjugate of Formula (I), 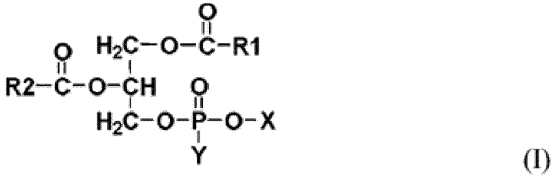 wherein: one of R1 and R2 is -L-carboranyl, and the other is a linear alkyl or alkenyl with 10-24 carbon atoms, wherein L is a divalent linking group; X is -H, - CH₂CH₂NH₃, -CH₂CH₂N⁺(CH₃)₃, -CH₂CH(NH₂)COOH, -CH₂CH(OH)CH₂OH, or 2,3,4,5,6-pentahydroxy-cyclohexan-1-yl; and Y is OH or O⁻.

In the carborane-phospholipid conjugate of the present disclosure, carborane is introduced to a lipid arm of phospholipid so that it has a structure similar to that of phospholipid in cells and thus has good compatibility with cells.

Carborane is a class of polyhedral boron-carbon molecular cluster compound. In the present application, the carboranyl refers to a group derived by removing a hydrogen atom from C-H bond of carborane.

In an embodiment of the present disclosure, the carboranyl is derived by removing a hydrogen atom from the C-H bond of the following carboranes: 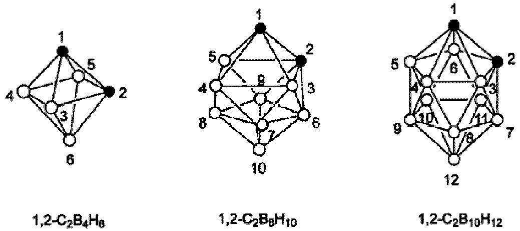 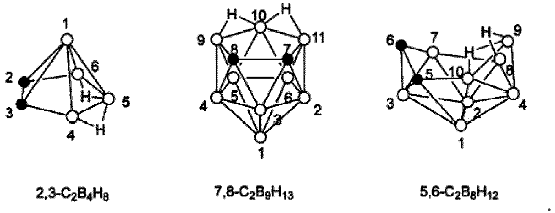 In other words, the carboranyl is 1,2-C₂B₄H₅-, 1,2-C₂B₈H₉-, 1,2-C₂B₁₀H₁₁-, 2,3-C₂B₄H₇-, 7,8-C₂B₉H₁₂- or 5,6-C₂B₈H₁₁-.

In a preferred embodiment, the carboranyl is derived by removing a hydrogen atom from the C-H bond of a carborane containing 8 to 11 boron atoms. In a more preferred embodiment, the carboranyl is derived by removing a hydrogen atom from the C-H bond of dicarba-closo-dodecaborane (C₂B₁₀H₁₂), *i.e.* 1,2-C₂B₁₀H₁₁-.

The boron atom in the carborane/carboranyl in the present application can be composed of naturally abundant boron element, or can be ¹⁰B-enriched. In an embodiment, one or more boron atoms in the carborane/carboranyl group are ¹⁰B-enriched. For example, the carborane/carboranyl has a ¹⁰B abundance of more than 20%, 30%, 40%, 50%, 60%, 70%, 80%, 90%, 95%, 96%, or 97%, or even more than 98% or 99%. The ¹⁰B-enriched carborane can be prepared using ¹⁰B-enriched boronic acid as raw material, or can be commercially available from Katchem spol. s r. o., Czech Republic (http//: www.katchem.cz/en).

In an embodiment, at least one boron atom in the carborane/carboranyl is ¹⁰B.

In an embodiment, the boron atoms in the carborane-phospholipid conjugate are ¹⁰B-enriched. For example, at least one boron atom in the carborane-phospholipid conjugate is ¹⁰B.

In an embodiment, one or more boron atoms in the ¹⁰B-enriched conjugate of General Formula (I) have ¹⁰B abundance of more than 30%, 40%, 50%, 60%, 70%, or 80%, preferably more than 90%, 95%, 96%, or 97%, or even more preferably more than 98% or 99%. It should be understood that each boron atom can have the same or different ¹⁰B abundance as those of other boron atoms.

In an embodiment, each boron atom in the ¹⁰B-enriched conjugate of General Formula (I) has a ¹⁰B abundance of more than 90%.

The carborane-phospholipid conjugates, in which X is -H, -CH₂CH₂NH₃, - CH₂CH₂N⁺(CH₃)₃, -CH2CH(NH2)COOH, -CH₂CH(OH)CH₂OH, or 2,3,4,5,6-pentahydroxy-cyclohexan-1-yl, are derivatives of phosphatidic acid, phosphatidylethanolamine, phosphatidylcholine, phosphatidylserine, phosphatidylglycerol, or phosphatidylinositol, respectively.

In an embodiment, X is -CH₂CH₂N⁺(CH₃)₃; Y is O⁻, that is, the carborane-phospholipid conjugate is a phosphatidylcholine derivative.

In the present application, the difference in length between the lipid arms refers to a distance from the carboxyl C at the head of R1 to the non-H atom at the end of the tail of R1 minus a distance from the carboxyl C of the head of R2 to the non-H atom at the end of the tail of R2.

In the present application, the distance between the lipid arms is a distance between the first C in R1 close to the glycerol group and the first C in R2 close to the glycerol group in the compound of Formula (I).

In the present application, the dihedral angle between the lipid arms refers to a dihedral angle formed by the non-H atom at the end of the tail of R1, the carboxyl C at the head of R1, the carboxyl C at the head of R2, and the non-H atom at the end of the tail of R2.

In an embodiment of the carborane-phospholipid conjugate of the present disclosure, the two lipid arms each comprising R1 or R2 satisfy one, two or three of conditions of: a difference in length within ± 3A; a distance between the lipid arms within 15A; and a dihedral angle within 15 degrees.

In an embodiment of the carborane-phospholipid conjugate of the present disclosure, the two lipid arms each comprising R1 or R2 satisfy one, two or three of conditions of: a difference in length within ± 2A; a distance between the lipid arms within 10A; and a dihedral angle within 10 degrees.

In a preferred embodiment of the carborane-phospholipid conjugate of the present disclosure, the two lipid arms each comprising R1 or R2 satisfy one, two or three of conditions of: a difference in length within ± 1A; a distance between the lipid arms within 5A; and a dihedral angle within 5 degrees. In a more preferred embodiment of the disclosed carborane-phospholipid conjugates, it is preferred to have all the three at the same time.

One of R1 and R2 is a linear alkyl or alkenyl having 10 to 24 carbon atoms. In an embodiment, the linear alkyl or alkenyl contains 11, 13, 15, 17, 19, 21, or 23 carbon atoms.

The term "alkyl" as used herein refers to a linear or branched saturated hydrocarbon chain group consisting only of *n* carbon atoms and *2n*+*1* hydrogen atoms. For example, C₁₀-C₂₄ alkyl refers to a linear or branched saturated hydrocarbon chain group having 10 to 24 carbon atoms. A numerical range such as "10 to 24" refer to each integer within the given range. For example, "10 to 24 carbon atoms" means that the alkyl contains 10, 11, 12, 13, 14, 15, 16, 17, 18, 19, 20, 21, 22, 23 or 24 carbon atoms. The linear alkyl refers to an unbranched alkyl group, such as decyl (C₁₀H₂₁-), lauryl (C₁₂H₂₅-), myristyl (C₁₄H₂₉-), palmityl (C₁₆H₃₃-), stearyl (C₁₈H₃₇-), eicosyl (C₂₀H₄₁-), behenyl (C₂₂H₄₅-), or tetracosyl (C₂₄H₄₉-) .

In the present application, the alkenyl refers to a linear or branched hydrocarbon chain group consisting only of carbon and hydrogen atoms and containing at least one double bond (*e*.*g*., 1 or 2 double bonds), *e.g., cis-9-*tetradecenyl, *trans*-9-tetradecenyl, *cis*-9-hexadecenyl, *trans*-9-hexadecenyl, *cis-9-*octadecenyl, *trans*-9-octadecenyl, *cis*-11-octadecenyl, *cis*,*cis*-9,12-octadecadienyl, *trans*,*trans*-9,12-octadecadienyl, and *cis*-13-docosenyl, etc. The linear alkenyl refers to an unbranched alkenyl.

L is a divalent linking group. In a preferred embodiment, L is a group with flexible hydrophobic chain. In a more preferred embodiment, L has comparable hydrophobicity and flexibility to those of the carborane-free lipid arm in the carborane-phospholipid conjugate. For example, L is -(CH₂)ₙ₊ₘ-, -(CH₂)ₙ₊ₘ-S-, - (CH₂)ₙ₊ₘ-O-, -(CH₂)ₙ-CH=CH-(CH₂)ₘ-, -(CH₂)ₙ-CH=CH-(CH₂)ₘ-S-, -(CH₂)ₙ-CH=CH-(CH₂)ₘ-O-, -(CH₂)ₙ-S-(CH₂)ₘ-, -(CH₂)ₙ-S-(CH₂)ₘ₊₁-S-, -(CH₂)ₙ-O-(CH₂)ₘ-, -(CH₂)ₙO-(CH₂)ₘ₊₁-O-, -(CH₂)ₙ-N(CH₃)-(CH₂)ₘ-, or -(CH₂)ₙ-N(CH₃)-(CH₂)ₘ₊₁-N(CH₃)-, wherein n is an integer from 1 to 20, and m is an integer from 0 to 20. In a specific embodiment, L is -(CH₂)ₙ₊ₘ-S-, wherein n is an integer from 1 to 20 and m is an integer from 0 to 20. For example, L is -(CH₂)₁₂-S-, -(CH₂)₁₃- S-, -(CH₂)₁₄-S-, -(CH₂)₁₅-S-, -(CH₂)₁₆-S-, -(CH₂)₁₇-S-, -(CH₂)₁₈-S-, -(CH₂)₁₉-S-, - (CH₂)₂₀-S-, -(CH₂)₂₁-S-, -(CH₂)₂₂ -S-, -(CH₂)₂₃-S-, or -(CH₂)₂₄-S-.

In a preferred embodiment, L is unbranched.

In a preferred embodiment of the present disclosure, the carborane-phospholipid conjugate has a structure of: 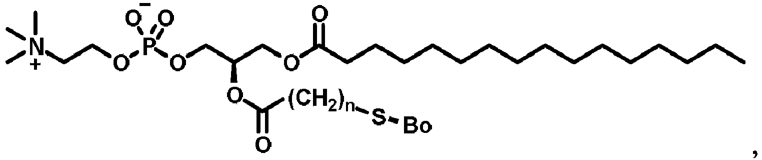 wherein Bo represents carboranyl, and n is an integer from 1 to 20. In a more preferred embodiment of the present disclosure, Bo is 1,2-C₂B₁₀H₁₁-, preferably dicarba-closo-dodecylboranyl, more preferably 1,2-dicarba-closo-dodecylboranyl; and n is 3, 7, 11, or 15, preferably 11.

### Synthesis of Carborane-Phospholipid Conjugate

The carborane-phospholipid conjugate of the present disclosure can be synthesized by a reaction of a C-substituted carborane derivative (*e*.*g*., carboranyl-L-COOH) with a lysophospholipid under the action of a dehydrating agent (*e*.*g*., dicyclohexylcarbodiimide (DCC)) in the presence of catalyst (*e*.*g*., 4-dimethylaminopyridine (DMAP)).

### Synthesis of C-Substituted Carborane Derivatives

The carborane cage has a strong electron-withdrawing property to make the H in the C-H bond weakly acidic, which can react with a strong base such as alkyl lithium reagent to prepare a series of C-substituted carborane derivatives. For example, carborane can react with oxetane, ethylene oxide, or formaldehyde under the action of butyllithium to introduce -CH₂CH₂CH₂OH, -CH₂CH₂OH, or -CH₂OH group to the carborane, respectively.

Carboranyl-L-COOH can be obtained by the oxidization of carboranyl-L-CH₂OH with an oxidizing agent, such as CrO₃.

The carborane derivative can be mono-substituted or di-substituted. In a preferred embodiment, the prepared carborane derivative is mono-substituted.

The carborane derivative can be synthesized using *tert*-butyldimethylsilyl (TBDMS) or triphenylsilyl as the C-terminal protecting group. For example, the mono- or di-substituted carborane derivative can be synthesized using triphenylsilyl as the C-terminal protecting group.

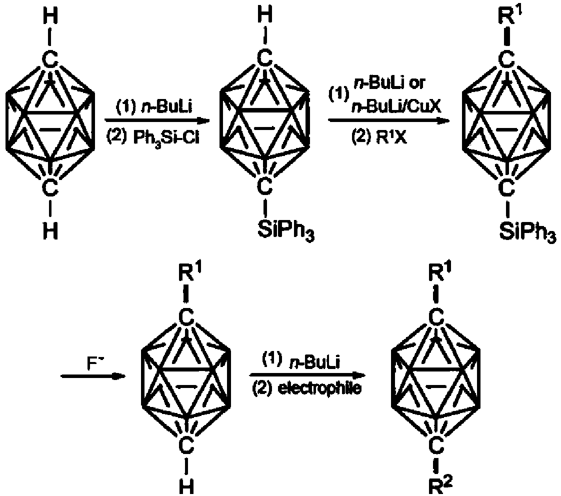

The synthesis of the C-substituted carborane derivative can be found in the review article of Lu, Juyou et al., Research Progress on Synthesis of Carborane Derivatives, Synthetic Chemistry, 2015, 23(9): 883 and He Y T, Ager J, Clark S, et al., A New Series of Organoboranes. III. Some Reactions of 1,2-Dicarbaclovododecaborane (12) and its Derivatives, Inorganic Chemistry, 1963, 2: 1097-1105.

### Synthesis of Lysophospholipid

Commercially available or housemade lysophospholipids can be used in the synthesis method of the present disclosure. The lysophospholipid can be prepared by enzymatic hydrolysis of a phospholipid, wherein the 1-position acyl is hydrolyzed with phospholipase A₁, and the 2-position acyl group is hydrolyzed with phospholipase A₂. The lysophospholipid used can comprise lysophosphatidylcholine, lysophosphatidic acid, lysophosphatidylethanolamine, lysophosphatidylserine, lysophosphatidylinositol, or the like. In an embodiment, the lysophospholipid used is lysophosphatidylcholine (Lyso-PPC).

The specific method for preparing the lysophospholipid can be found in the review article by Li, Zhao et al., China Oils and Fats, 2018, 43(6): 132-143. The phospholipid for use in hydrolysis can be commercially available or synthesized as needed.

The synthesis of carborane-phospholipid conjugate is illustrated below by taking Compounds BoPs 1-4 as example.

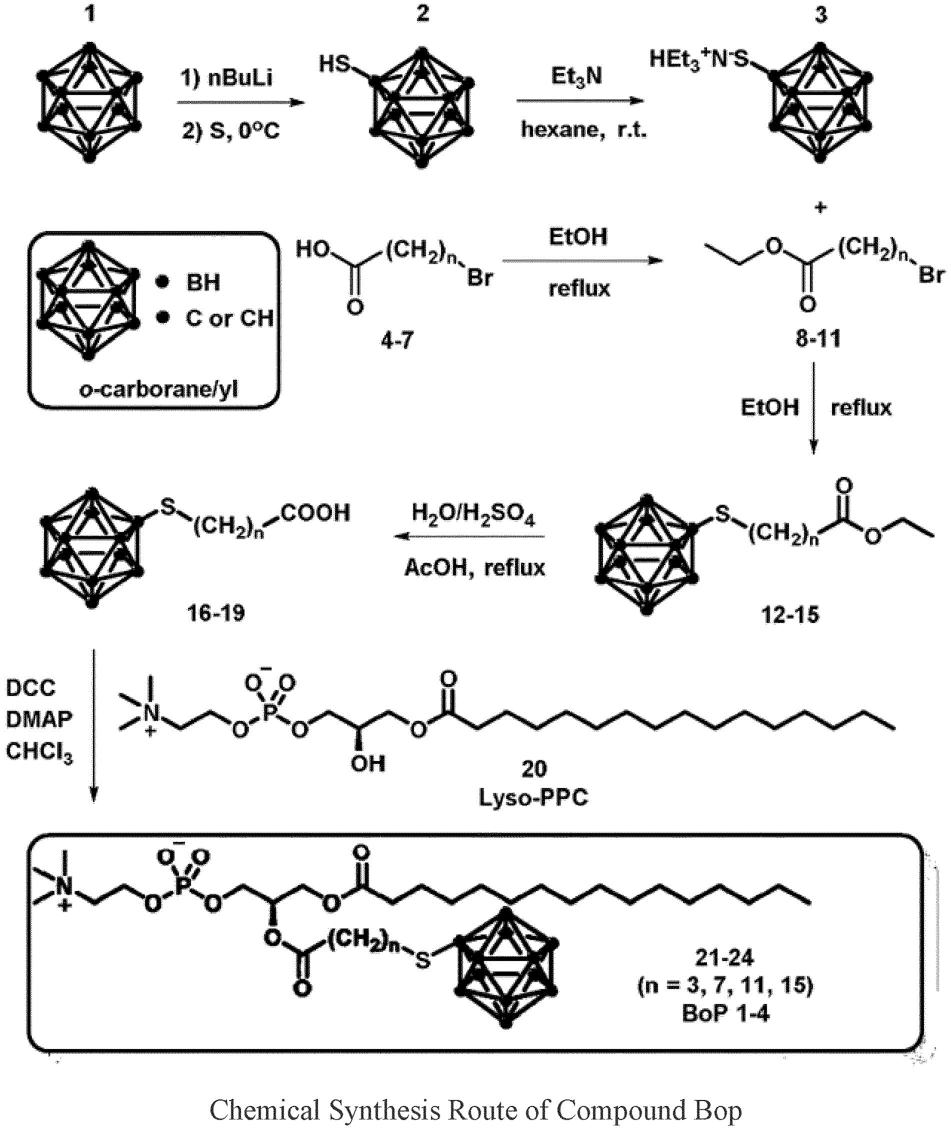

### Liposomes

Another aspect of the present disclosure provides a liposome composition comprising the carborane-phospholipid conjugate, wherein the carborane-phospholipid conjugate is a part of a lipid bilayer.

The liposome of the present disclosure can be a unilamellar liposome that is composed of a single lipid bilayer and has typically a diameter ranging from about 20 nm to about 400 nm. The liposome of the present disclosure can also be a multilamellar liposome, which has typically a diameter ranging from 1 µm to 10 µm.

Due to their cell membrane-like structure, the liposome of the present disclosure has a high affinity to cells and can increase the permeability of active ingredients (therapeutic agents and/or diagnostic agents) to cells so that the concentration and effect of the active ingredients are improved at the site of disease. The active ingredients are encapsulated in the liposome, and thus the diffusion rate in tissues is reduced and the release rate in the blood is slowed down, which prolonging the action time of the active ingredients. Under the protection of the liposome bilayer, the active ingredients can avoid oxidation, degradation, or destruction by acids or enzymes in the human body to ensure or prolong the stability of the active ingredients.

In an embodiment of the present disclosure, the liposome composition further comprises cholesterol. It is believed that cholesterol can reinforce the lipid bilayer membrane, reduce the fluidity of the phospholipid bilayer membrane, reduce the membrane permeability, and reduce the leakage of effective ingredients. At the same time, it can allow the lipid membrane to maintain a flexibility and enhance the ability of the liposome vesicle to resist changes in external conditions. In addition, it has a certain protective effect on the oxidation of phospholipids.

In an embodiment of the present disclosure, the liposome composition further comprises a phospholipid, preferably a saturated phospholipid. Examples of the phospholipid can comprise dilauroylphosphatidylcholine (DLPC), dimyristoylphosphatidylcholine (DMPC), distearoylphosphatidylcholine (DSPC), dioleoylphosphatidylcholine (DOPC), dipalmitoylphosphatidylcholine (DPPC), dimyristoylphosphatidylglycerol (DMPG), distearoylphosphatidylglycerol (DSPG), dioleoylphosphatidylglycerol (DOPG), dipalmitoylphosphatidylglycerol (DPPG), dimyristoylphosphatidylserine (DMPS), distearoylphosphatidylserine (DSPS), dioleoylphosphatidylserine (DOPS), dipalmitoylphosphatidylserine (DPPS), dioleoylphosphatidylethanolamine (DOPE), dipalmitoylphosphatidylethanolamine (DPPE), dimyristoylphosphatidylethanolamine (DMPE), distearoylphosphatidylethanolamine (DSPE), dididaidoylphosphatidylethanolamine, hydrogenated soybean phosphatidylcholine (HSPC) or the like, preferably DPPC. In a preferred embodiment, the phospholipid contained in the liposome composition has a similar structure to that of the phospholipid in the carborane-phospholipid conjugate of Formula I, that is, having the same or similar R1 or R2 (*e*.*g*., having a carbon chain with length difference within 1) and the same or similar X and Y. It is believed that the addition of phospholipids can increase the stability of the bilayer lipid molecule and reduce the amount of the carborane-phospholipid conjugate.

In an embodiment of the present disclosure, the liposome composition further comprises a PEGylated phospholipid. The PEGylated phospholipid refers to a PEG-derivatized phospholipid formed by the covalent bonding between PEG molecule and phospholipid molecule. It is believed that PEG-derivatized phospholipids can decrease the leakage rate of liposomes, reduce their aggregation and fusion, prolong the effective period, inhibit cell adhesion in the systemic circulation, shield the recognition and uptake of liposomes by RES, and prolong the circulation time of liposomes in the body. The PEG in the PEGylated phospholipid has typically a molecular weight of about 500 to about 5,000 Daltons (Da; g/mol), such as a molecular weight of 750 Da, 1,000 Da, 2,000 Da, or 5,000 Da. The PEG in the PEGylated phospholipid can have a linear or branched chain structure.

In an embodiment of the present disclosure, the PEGylated phospholipid is preferably DLPE-PEG, DPPE-PEG, DMPE-PEG, or DSPE-PEG, more preferably DSPE-PEG, such as DSPE-PEG2000 and DSPE-PEG5000.

In an embodiment of the present disclosure, the liposome composition comprises both cholesterol and phospholipids.

In an embodiment of the present disclosure, the liposome composition comprises all of cholesterol, phospholipid, and PEGylated phospholipid. In a preferred embodiment of the present disclosure, the liposome composition comprises all of cholesterol, phospholipid, and PEGylated phospholipid, wherein the lipid arms in the phospholipid and the PEGylated phospholipids differ by 0, 1, or 2 carbon atoms.

The liposome in the present disclosure can further comprise a pharmaceutically acceptable formulation auxiliary, such as, stabilizer, antioxidant, pH adjuster, flavoring agent, carrier, diluent, excipient, or the like. The term "pharmaceutically acceptable" means that the formulation auxiliary should be necessarily compatible with other ingredients of the formulation and not deleterious to the subject receiving the formulation. In the present disclosure, the subject refers to a mammal, which can be human or pet.

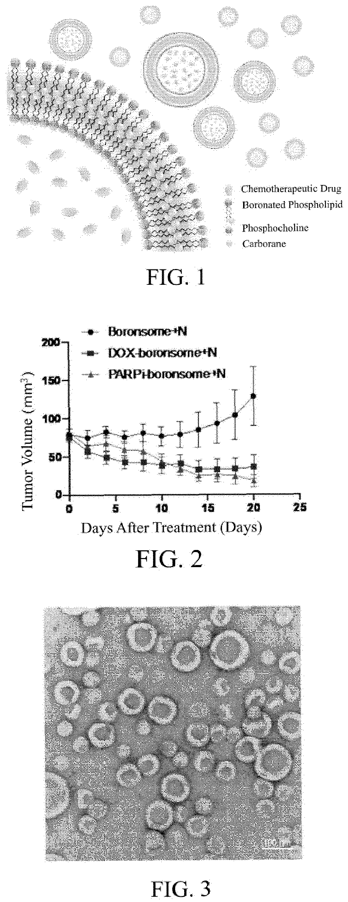

In an embodiment of the present disclosure, the liposome composition further comprises at least one therapeutic agent and/or at least one diagnostic agent. The therapeutic or diagnostic agent can be encapsulated within the water compartment of the liposome, or can be embedded into the lipid bilayer. As shown in FIG. 1, a water-soluble chemotherapeutic drug can be encapsulated within the water compartment of the liposome formed by a boronated phospholipid.

Suitable molecules for use as therapeutic agent comprise, but are not limited to, polypeptides, oligopeptides, peptidomimetics, amino acids, enzyme inhibitors, hormones, toxins, antibiotics, anti-inflammatory substances, anti-cancer drugs, immunosuppressants, bronchodilators, and the like.

In a preferred embodiment, the liposome composition comprises at least one therapeutic agent, which is an anti-cancer drug.

The therapeutic agent may be, *e.g.*, antimetabolites (*e*.*g*., antifolics (*e*.*g*., methotrexate), fluoropyrimidines (*e*.*g*., 5-fluorouracil), purine and adenosine analogs, cytarabine), embedded antitumor antibiotics (*e.g.*, anthracyclines (*e.g.*, doxorubicin, daunorubicin, epirubicin, idarubicin, mitomycin-C, dactinomycin, and mithramycin)), platinum derivatives (*e*.*g*., cisplatin and carboplatin), alkylating agents (*e*.*g*., nitrogen mustard, melphalan, chlorambucil, busulfan, cyclophosphamide, ifosfamide, nitrosoureas, thiotepa), antimitotics (*e*.*g*., vinca alkaloids (*e*.*g*., vincristine) and taxanes (*e*.*g*., Taxol^{®} (paclitaxel), Taxotere^{®} (docetaxel)), and more innovative microtubule agent (*e*.*g*., epothilone analogs, discodermolide analogs, and eleuterobin analogs)), topoisomerase inhibitors (*e*.*g*., epipodophyllotoxins (*e*.*g*., etoposide, teniposide, Amsacrine, and topotecan)), cell cycle inhibitors (*e*.*g*., flavopyridol), biological response modifiers, and proteasome inhibitors (*e*.*g*., Velcade^{®} (bortezomib)).

For example, the anti-cancer drug is selected from the group consisting of monomethyl auristatin E, monomethyl auristatin F, ibrutinib, acalabrutinib, zanubrutinib, doxorubicin, mitomycin-C, mitomycin-A, daunorubicin, aminopterin, actinomycin, bleomycin, 9-aminocamptothecin, N8-acetylspermidine, 1-(2-chloroethyl)-1,2-dimethanesulfonylhydrazide, Yunnanmycin, gemcitabine, cytarabine, dolastatin, dacarbazine, 5-fluorouracil; paclitaxel, docetaxel, gemcitabine, cytarabine, 6-mercaptopurine, vincristine, cisplatin, oxaliplatin, PARP inhibitors.

In an embodiment, the anti-cancer drug is selected from the group consisting of PARP inhibitors, preferably olaparib, niraparib, rucaparib, fluzoparib, pamiparib, veliparib, talazoparib, more preferably olaparib.

The liposome of the present disclosure can comprise a diagnostic agent. The diagnostic agent used in the present disclosure can comprise any diagnostic agent that is known in the art, *e*.*g*., those provided in Armstrong et al., Diagnostic Imaging, 5th Edition, Blackwell Publishing (2004); Torchilin, V.P. Ed., Targeted Delivery of Imaging Agents, CRC Press (1995); Vallabhajosula, S., Molecular Imaging: Radiopharmaceuticals for PET and SPECT, Springer (2009). The diagnostic agent can be detected by many means, including agents providing and/or enhancing a detectable signal, wherein the signal comprises, but is not limited to, gamma-emitting, radioactive, echogenic, optical, fluorescent, absorption, magnetic, or tomographic signals.

The technique for imaging the diagnostic agent can comprise, but is not limited to, single photon emission computed tomography (SPECT), magnetic resonance imaging (MRI), optical imaging, positron emission tomography (PET), computed tomography (CT), X-ray imaging, gamma-ray imaging, etc. The diagnostic agent can be associated with the therapeutic liposomes by various ways, including being embedded or encapsulated into the liposomes, for example.

In some embodiments, the diagnostic agent can comprise a chelating agent that binds to a metal ion for use in a variety of diagnostic imaging techniques. Exemplary chelating agents comprise, but are not limited to, ethylenediaminetetraacetic acid (EDTA), [4-(1,4,8,11-tetraazacyclotetradecan-1-yl)methyl]benzoic acid (CPTA), cyclohexanediaminetetraacetic acid (CDTA), ethylene glycol bis(2-aminoethyl ether)tetraacetic acid (EGTA), diethylenetriaminepentaacetic acid (DTPA), citric acid, hydroxyethylethylenediaminetriacetic acid (HEDTA), iminodiacetic acid (IDA), triethylenetetraminehexaacetic acid (TTHA), 1,4,7,10-tetraazacyclododecane-1,4,7,10-tetra(methylene phosphate) (DOTP), 1,4,8,11-tetraazacyclododecane-1,4,8,11-tetraacetic acid (TETA), 1,4,7,10-tetraazacyclododecane-1,4,7,10-tetraacetic acid (DOTA), and their derivatives.

A radioisotope can be incorporated into some of the diagnostic agents as described herein and can comprise a radionuclide that emits gamma ray, positron, beta- and alpha-particles, and X-ray. Suitable radionuclides comprise, but are not limited to, ²²⁵Ac, ⁷²As, ²¹¹At, ¹¹B, ¹²⁸Ba, ²¹²Bi, ⁷³Br, ⁷⁷Br, ¹⁴C, ¹⁰⁹Cd, ⁶²Cu, ⁶⁴Cu, ⁶⁷Cu, ¹⁸F, ⁶⁷Ga, ⁶⁸Ga, ³H, ¹²³I, ¹²⁵I, ¹³⁰I, ¹³¹I, ¹¹¹In, ¹⁷⁷Lu , ¹³N , ¹⁵O , ³²P, ³³P, ²¹²Pb, ¹⁰³Pd, ¹⁸⁶Re, ¹⁸⁸Re, ⁴⁷Sc, ¹⁵³Sm, ⁸⁹Sr, ^{99m}Tc, ⁸⁸Y, and ⁹⁰Y. In certain embodiments, the radioactive reagents can comprise ¹¹¹In-DTPA, ^{99m}Tc(CO)₃-DTPA, ^{99m}Tc(CO)₃-ENPy₂, ^{62/64/67}Cu-TETA , ^{99m}Tc(CO)₃-IDA, and ^{99m}Tc(CO)₃ triamine (circular or linear). In other embodiments, the reagents can comprise DOTA and its analogs having ¹¹¹In, ¹⁷⁷Lu, ¹⁵³Sm, ^{88/90}Y, ^{62/64/67}Cu, or ^{67/68}Ga. In some embodiments, the liposome can be radiolabeled, e.g., by incorporating a lipid associated with a chelating agent, such as, DTPA-lipid, such as, those provided in Phillips et al., Wiley Interdisciplinary Reviews: Nanomedicine and Nanobiotechnology, 1(1):69-83(2008); Torchilin, V.P. & Weissig, V. Eds., Liposomes 2nd Edition: Oxford Univ. Press(2003); Elbayoumi, T.A. & Torchilin, V.P., Eur. J. Nucl. Med. Mol. Imaging 33:1196-1205(2006); Mougin-Degraef, M. et al., Int'l J. Pharmaceuticals 344:110-117(2007).

In other embodiments, the diagnostic agent can comprise an optical agent such as fluorescent agent, phosphorescent reagent, chemiluminescent reagent, or the like. A variety of reagents (*e*.*g*., dyes, probes, labels, or indicators) are known in the art and can be used in the present invention (see, *e*.*g*., Invitrogen, The Handbook - A Guide to Fluorescent Probes and Labeling Technologies, 10th Edition (2005)). The fluorescent agent can comprise a variety of organic and/or inorganic small molecules, or a variety of fluorescent proteins and derivatives thereof. For example, the fluorescent agent can comprise, but is not limited to, cyanine, phthalocyanine, porphyrin, indigo, rhodamine, phenoxazine, phenylxanthene, phenothiazine, phenoselenazine, fluorescein, benzoporphyrin, squaryamine, dipyrrolopyrimidone, tetracenes, quinoline, pyrazine, corrin, croconiums, acridone, phenanthridine, rhodamine, acridine, anthraquinone, chalcogenopyrylium analogs, chlorins, naphthalocyanine, methinedyes, indoleniumdyes, azo-compounds, azulene, azaazulene, triphenylmethane dyes, indole, benzoindole, indocarbocyanine, benzindocarbocyanine, and BODIPY TM derivatives with general structure of 4,4-difluoro-4-boron-3a,4a-diaza-s-dicyclopentacene, and/or any conjugates and/or derivatives thereof. Other reagents that can be used comprise, but are not limited to, *e*.*g*., fluorescein, fluorescein-polyaspartate conjugates, fluorescein-polyglutamic acid conjugates, fluorescein-polyarginine conjugates, indocyanine green, indigo-dodecaspartic acid conjugate, indigo-polyaspartic acid conjugate, isosulfan blue, indoledisulfonates, benzoindole disulfonates, bis(ethylcarboxymethyl)indigo, bis(pentylcarboxymethyl)indigo, polyhydroxyindole sulfonate, polyhydroxybenzoindole sulfonate, rigid heteroatom indole sulfonate, indigo dipropionic acid, indigo dicaproic acid, 3,6-dicyano-2,5-[(N,N,',N'-tetrakis(carboxymethyl)amino]pyrazine, 3,6-[(N,N,',N'-tetrakis(2-hydroxyethyl)amino]pyrazine-2,5-dicarboxylic acid, 3,6-bis(N-azatedino)pyrazine-2,5-dicarboxylic acid, 3,6-bis(N-morpholino)pyrazine-2,5-dicarboxylic acid, 3,6-bis(N-piperazino)pyrazine-2,5-dicarboxylic acid, 3,6-bis(N-thiomorpholino)pyrazine-2,5-dicarboxylic acid, 3,6-bis(N-thiomorpholino)pyrazine-2,5-dicarboxylic acid S-oxide, 2,5-dicyano-3,6-bis(N-thiomorpholino)pyrazine S,S-dioxide, indocarbocyaninetetrasulfonate, chloroindocarbocyanine, and 3,6-diaminopyrazine-2,5-dicarboxylic acid.

In a preferred embodiment of the present disclosure, the liposome composition comprises at least one diagnostic agent, which is ⁶⁴Cu-NOTA-PEG2000-DSPE.

The liposome of the present disclosure can be prepared by conventional methods in the art, such as, injection method (ethanol injection method and ether injection method), film dispersion method, reverse phase evaporation method, chemical gradient method (pH gradient method, ammonium sulfate gradient method, calcium acetate gradient method), multiple emulsion method, freeze-drying method and other method, see Chinese Pharmaceutical Journal, Volume 46, Issue 14, Pages 1084-1088, 2011.

In an embodiment, a blank liposome comprising *aq*. ammonium sulfate is prepared by ethanol injection method or film dispersion method, then the ammonium sulfate outside the liposome membrane is removed to form a gradient of ammonium sulfate, and a therapeutic- or diagnostic-agent-loaded liposome is formed by ammonium sulfate gradient method.

The particle size of the liposome of the present disclosure can be measured by thermodynamic light scattering method, *e*.*g*., using the Zetasizer 3000SH laser particle size analyzer (Malvern Instruments Ltd). The diameter is mainly about 20 to about 150 nm.

The encapsulation efficiency of the liposome in the present disclosure can be measured by centrifugation, dialysis, or gel column chromatography methods, etc.

The liposome according to the present disclosure can be administered orally or non-orally (*e*.*g*., those suitable for intravenous, subcutaneous, intraperitoneal or local administration) as needed. The amount of administration varies depending on the patient's weight, age, sex, health status, diet, time of administration, method of administration, excretion r transpulmononary ate, and severity of the disease. For example, the liposome of the present disclosure can be administered by intravenous, pulmonary (aerosol inhalation or dry powder inhalation), or dermal routes as needed. The amount of administration is approximately 0.1 to 10 mg/kg weight based on the weight of the therapeutic agent or diagnostic agent contained in the liposome.

### EXAMPLE

The starting materials for the examples are commercially available and/or can be prepared by a variety of methods which are well known to those skilled in the field of organic synthesis. Those skilled in the field of organic synthesis will suitably select reaction conditions (including solvent, reaction atmosphere, reaction temperature, duration of the experiment, and post-treatment) in the following synthesis methods. Those skilled in the art of organic synthesis will understand that the functional groups present on various moieties of the molecule should be compatible with the reagents and reactions proposed. NMR is recorded using a Bruker AVANCE 400 MHz spectrometer. High-resolution mass spectrometry is measured using a Bruker Fourier Transform Ion Cyclotron resonance mass spectrometer. The liquid chromatography-mass spectrometry used is Waters e2695 instrument equipped with Waters 2995 PDA and Waters Acquity QDA mass spectrometer.

### 1. Chemical Synthesis of BoP Series

A series of BoPs (BoPs 1-4) were synthesized using the above synthetic scheme, wherein the number of methylene in the carbon chain, *n,* is 3, 7, 11, 15. Individual compounds were arranged in accordance with their numbers from small to large, and had a total yield of 11% to 20 % and a purity > 97%.

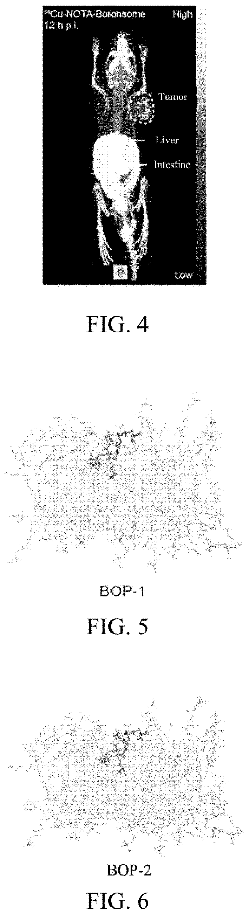

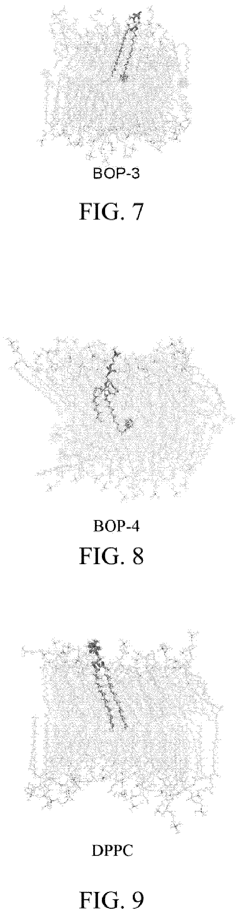

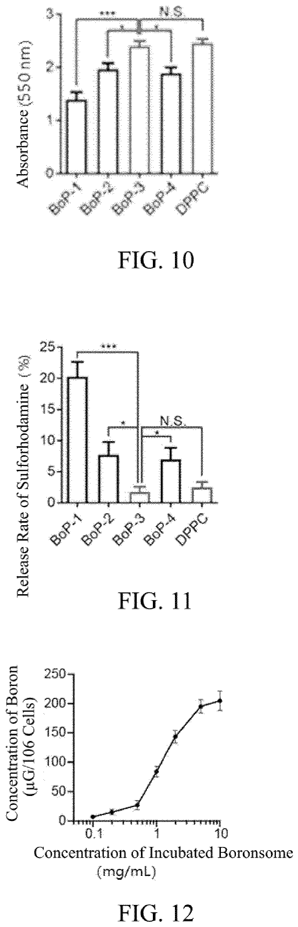

The specific synthesis steps and characterized methods of BoP-3 were as follows:
(1) Chemical Synthesis of Carborane Mercaptotriethylamine Salt **(1):**
   A substrate (o-carborane, 1.00 g, 6.93 mmol) was dissolved in THF (30 mL), and cooled to 0°C under anhydrous and anaerobic conditions. Then, n-butyllithium (4.4 mL, 6.93 mmol) was added dropwise to the solution for 15 min, and stirred for 30 min. Then, the system was warmed to room temperature for 30 min. Sublimated sulfur (225 mg, 6.93 mmol) was added and reacted in an ice-water bath for 30 min. Then, the system was warmed to room temperature for 30 min. The solution was evaporated to dryness with rotation to give a yellow liquid. Then, hydrochloric acid (1 M, 7 mL) was added to quench the unreacted *n-*butyllithium. The solution was extracted with *n*-hexane, washed with water and saturated brine successively, and evaporated to dryness with rotation . To a solution of the product in *n*-hexane (6 mL) was added dropwise triethylamine (1 mL, 7.18 mmol) at room temperature, and the mixture was stirred for 15 min. A white precipitate was formed, filtered by suction, washed with 3x *n*-hexane (6 ml), and dried in the air.
(2) Preparation of Carboranized Fatty Acid: To a solution of 1-HOOC(CH₂)₁₁Br (9.3 mmol) in ethanol (50 mL) was added dropwise 5 mL of concentrated sulfuric acid. The reaction mixture was stirred at room temperature for 15 min, and heated to reflux for 12 h. The mixture was cooled and evaporated to dryness with rotation . Ethyl acetate (30 mL) and water (30 mL) were added for extraction. The organic phase was washed with pure water and saturated brine, dried over anhydrous Na₂SO₄, and evaporated to dryness with rotation . The crude product was purified by column chromatography on silica gel column with petroleum ether and ethyl acetate as eluent. The eluate was evaporated to dryness with rotation under reduced pressure to give I-C₂H₅OC(O)(CH₂)₁₁Br. To a solution of **1** (1.86 mmol) in ethanol (42 mL) was added Br(CH₂)₁₁C(O)OC₂H₅ (1.86 mmol). The reaction mixture was stirred at room temperature for 15 min, and heated to react for 16 h. The reaction mixture was cooled, and evaporated to dryness with rotation . The residue was extracted with ethyl acetate (30 mL), and washed with pure water (30 mL). The organic phase was separated, washed with water and saturated brine, dried over anhydrous Na₂SO₄, and evaporated to dryness with rotation . The crude product was purified by column chromatography on silica gel column. The eluate was evaporated to dryness with rotation under reduced pressure to remove solvent, to give 1-C₂H_{S}OC(O)(CH₂)₁₁S-1,2-C₂B₁₀H₁₁. The product was dissolved in glacial acetic acid (29 mL) to form a colorless clear solution. The mixture was stirred at room temperature for 15 min, and pure water (9.8 mL) and concentrated sulfuric acid (9.8 mL) were sequentially added dropwise to hydrolyze for 16 h to give 1-HOOCC(O)(CH₂)₁₁S-1,2-C₂B₁₀H₁₁ (**2**).
(3) Preparation of BoP-3: To a solution of 0.1 M carboranyl fatty acid (with carbon atom number of 12, **2)** (2.0 *eq.*) were added dicyclohexylcarbodiimide (DCC, 2.6 *eq.*) and 4-dimethylaminopyridine (DMAP, 4.0 *eq.*) in chloroform. The solution was stirred at room temperature for 5 min. Then, Lyso-PPC (1.3 *eq.*) was added, and the reaction was stirred for 24 hours. The reaction mixture was concentrated by rotary evaporation, and purified by silica gel column chromatography (65:25:4 CH2Cl₂:MeOH:H₂O) to give BoP-3.
(4) Characterization of BoP-3: BoPs with different numbers of carbon atoms (BoP-1, BoP-2, BoP-3, BoP-4) were observed for the conformations of membrane structure formed thereby via molecular dynamics simulation, and it was found that BoP-3 has the most stable conformation, the minimal folding degree of long chain, and the closest membrane structure to that formed by DPPC (FIG. 5 to FIG. 9). At the same time, they were further tested for their drug loading efficiency by encapsulating sulforhodamine B (SRB) and measuring the absorption at 550 nm, and it was found that BoP-3 had the highest absorption (FIG. 10), indicating the most intact membrane structure. Also, they were measured for their SRB leakage, and it was found that after incubation in 50% bovine serum at 37°C for 24 hours, BoP-3 had the lowest SRB leakage percentage (FIG. 11). At the same time, they were further characterized by means of hydrogen nuclear magnetic resonance spectroscopy (¹H NMR) to show the correct structures. To more intuitively obtain information on the membrane structure formed by BoP-3, a boronsome composed of BoP-3, DPPC, cholesterol and DPPE-PEG2000 was prepared. We found that via particle control by extruder and purification by dialysis, the boronsome were produced as spherical carriers which were 30-100 nm in diameter, had regular shapes, and were almost no damage (see FIG. 3), as observed by transmission electron microscopy (TEM). Also, the spherical carriers were measured for their polydispersity index and zeta potential. In the case of encapsulating doxorubicin (Dox), the electrical neutrality reached -3.1 mV, indicating an effective drug loading performance.

### Preparation of BoP-1, BoP-2, BoP-4:

BoP-1, BoP-2, BoP-4 and BoP-3 were prepared by substantially the same methods, except for the differences in the carbon chain lengths of the bromocarboxylic acids selected in the initial synthesis of carboranized fatty acids. Among them, the substrate was 1-HOOC(CH₂)₃Br for BoP-1, 1-HOOC(CH₂)₇Br for BoP-2, and 1-HOOC(CH₂)₁₅Br for BoP-4, respectively. The detailed procedure was as follows:
(1) BoP-1: To a solution of 1-HOOC(CH₂)₃Br (9.3 mmol) in ethanol (50 mL) was added dropwise 5 mL of concentrated sulfuric acid. The mixture was stirred at room temperature for 15 min, and heated to reflux for 12 h. The mixture was cooled, and evaporated to dryness with rotation . Ethyl acetate (30 mL) and water (30 mL) were added for extraction, and the organic phase was washed with pure water and saturated brine, dried over anhydrous Na₂SO₄, and rotary-evaporated to dryness. The crude product was purified by column chromatography on silica gel column with petroleum ether and ethyl acetate as eluent. The eluate was evaporated to dryness with rotation under reduced pressure to remove the solvent to give 1-C₂H₅OC(O)(CH₂)₃Br. To a solution of **1** (1.86 mmol) in ethanol (42 mL) was added Br(CH₂)₃C(O)OC₂H₅ (1.86 mmol). The mixture was stirred at room temperature for 15min, and was heated to reaction for 16 h. The reaction mixture was cooled, and evaporated to dryness with rotation . The residue was extracted with ethyl acetate (30 mL) and washed with pure water (30 mL). The organic phase was separated, washed with water and saturated brine, dried over anhydrous Na₂SO₄, and evaporated to dryness with rotation . The crude product was purified by column chromatography on silica gel column. The eluate was evaporated to dryness with rotation under reduced pressure to remove the solvent to give 1-C₂H₅OC(O)(CH₂)₃S-1,2-C₂B₁₀H₁₁. The resulting product was dissolved in glacial acetic acid (29 mL) to form a colorless clear solution. The solution was stirred at room temperature for 15 min, and pure water (9.8 mL) and concentrated sulfuric acid (9.8 mL) were added dropwise to hydrolyze for 16 h to give 1-HOOCC(O)(CH₂)₃S-1,2-C₂B₁₀H₁₁ **(3).** To a solution of 0.1 M carboranyl fatty acid (with the carbon number of 4, **3)** (2.0 *eq.*) were added dicyclohexylcarbodiimide (DCC, 2.6 *eq.*) and 4-dimethylaminopyridine (DMAP, 4.0 *eq.*) in chloroform. The solution was stirred at room temperature for 5 min. Lyso-PPC (1.3 *eq.*) was then added, and the reaction was stirred for 24 hours. The reaction mixture was concentrated by rotary evaporation, and purified by silica gel column chromatography (65:25:4 CH2Cl2:MeOH:H2O) to give BoP-1.
(2) BoP-2: To a solution of 1-HOOC(CH₂)₇Br (9.3 mmol) in ethanol (50 mL) was added dropwise 5 mL of concentrated sulfuric acid. The mixture was stirred at room temperature for 15 min, and heated to reflux for 12 h. The mixture was cooled, and evaporated to dryness with rotation. Ethyl acetate (30 mL) and water (30 mL) were added for extraction, and the organic phase was washed with pure water and saturated brine, dried over anhydrous Na₂SO₄, and evaporated to dryness with rotation. The crude product was purified by column chromatography on silica gel column with petroleum ether and ethyl acetate as eluent. The eluate was evaporated to dryness with rotation under reduced pressure to remove the solvent to give 1-C₂H₅OC(O)(CH₂)₇Br. To a solution of **1** (1.86 mmol) in ethanol (42 mL) was added Br(CH₂)₇C(O)OC₂H₅ (1.86 mmol). The reaction mixture was stirred at room temperature for 15 min, and then heated for 16 h. The reaction mixture was cooled, and evaporated to dryness with rotation. The residue was extracted with ethyl acetate (30 mL) and washed with pure water (30 mL). The organic phase was separated, washed with water and saturated brine, dried over anhydrous Na₂SO₄, and evaporated to dryness with rotation. The crude product was purified by column chromatography on silica gel column. The eluate was evaporated to dryness with rotation under reduced pressure to remove the solvent to give 1-C₂H₅OC(O)(CH₂)₇S-1,2-C₂B₁₀H₁₁. The resulting product was dissolved in glacial acetic acid (29 mL) to form a colorless and clear solution. The solution was stirred at room temperature for 15 min, and pure water (9.8 mL) and concentrated sulfuric acid (9.8 mL) were added dropwise to hydrolyze for 16 h to give 1-HOOCC(O)(CH₂)₇S-1,2-C₂B₁₀H₁₁ **(4).** To a solution of 0.1 M carboranyl fatty acid (with the carbon number of 8, **4)** (2.0 *eq.*) were added dicyclohexylcarbodiimide (DCC, 2.6 *eq.*) and 4-dimethylaminopyridine (DMAP, 4.0 *eq.*) in chloroform. The solution was stirred at room temperature for 5 min. Lyso-PPC (1.3 *eq.*) was then added, and the reaction was stirred for 24 hours. The reaction mixture was concentrated by rotary evaporation, and purified by silica gel column chromatography (65:25:4 CH2Cl2:MeOH:H2O) to give BoP-2.
(3) BoP-4: To a solution of 1-HOOC(CH₂)₁₅Br (9.3 mmol) in ethanol (50 mL) was added dropwise 5 mL of concentrated sulfuric acid. The mixture was stirred at room temperature for 15 min, and heated to reflux for 12 h. The mixture was cooled, and evaporated to dryness with rotation. Ethyl acetate (30 mL) and water (30 mL) were added for extraction, and the organic phase was washed with pure water and saturated brine, dried over anhydrous Na₂SO₄, and evaporated to dryness with rotation. The crude product was purified by column chromatography on silica gel column with petroleum ether and ethyl acetate as eluent. The eluate was evaporated to dryness with rotation under reduced pressure to remove the solvent to give 1-C₂H₅OC(O)(CH₂)₁₅Br. To a solution of **1** (1.86 mmol) in ethanol (42 mL) was added Br(CH₂)₁₅C(O)OC₂H₅ (1.86 mmol). The reaction was stirred at room temperature for 15 min, and heated for 16 h. The reaction mixture was cooled, and evaporated to dryness with rotation. The residue was extracted with ethyl acetate (30 mL) and washed with pure water (30 mL). The organic phase was separated, washed with water and saturated brine, dried over anhydrous Na₂SO₄, and evaporated to dryness with rotation. The crude product was purified by column chromatography on silica gel column. The eluate was evaporated to dryness with rotation under reduced pressure to remove the solvent to give 1-C₂H₅OC(O)(CH₂)₁₅S-1,2-C₂B₁₀H₁₁. The resulting product was dissolved in glacial acetic acid (29 mL) to form a colorless and clear solution. The solution was stirred at room temperature for 15 min, and pure water (9.8 mL) and concentrated sulfuric acid (9.8 mL) were added dropwise to hydrolyze for 16 h to give 1-HOOCC(O)(CH₂)₁₅S-1,2-C₂B₁₀H₁₁ **(4).** To a solution of 0.1 M carboranyl fatty acid (with the carbon number of 16, **5)** (2.0 *eq.*) were added dicyclohexylcarbodiimide (DCC, 2.6 *eq.*) and 4-dimethylaminopyridine (DMAP, 4.0 *eq.*) in chloroform. The solution was stirred at room temperature for 5 min. Lyso-PPC (1.3 *eq.*) was then added, and the reaction was stirred for 24 hours. The reaction mixture was concentrated by rotary evaporation, and purified by silica gel column chromatography (65:25:4 CH2Cl2:MeOH:H2O) to give BoP-4.

### 2. Composition and Preparation of Boronsome

Distearoylphosphatidylcholine (DPPC), cholesterol (Chol), distearoylphosphatidylethanolamine-polyethylene glycol 2000 (DSPE-PEG2k) and BoP with a total mass of 20 mg were dissolved in 1 mL of ethanol at 60°C at a molar ratio of BoP:DPPC:Chol:DSPE-PEG2k = 50:10:40:1, and then an ammonium sulfate buffer (4 mL, 250 mM, pH=5.5) was injected at 60°C. Then, the liposome solution was allowed to pass through a liposome extruder with membrane pore size of 200 nm at 60°C, and push was applied 10 times in total. Subsequently, the membrane was replaced by a membrane with membrane pore size of 100 nm, and push was applied for additional 10 times in total. Free ammonium sulfate was removed by dialysis in 800 mL of a solution consisting of 10% sucrose and 10 mM histidine (pH = 6.5), with the dialysate replaced at least twice within 12 hours.

### 3. Drug Loading of Boronsome

For sulforhodamine (SRB)-loaded boronsome, the lipid of the formulation was dissolved in ethanol, hydrated with 50 mM SRB, and sonicated at 45°C for 30 min. Doxorubicin (Dox) was loaded by ammonium sulfate gradient method. Dox was added to the liposome solution at a drug-to-lipid molar ratio of 1:8, and cultured at 60 ° C for 1 hour. The stability and encapsulation ability of the boronsome were evaluated by SRB release rate (% SRB release rate = (*A_{final}* - *Aᵢₙᵢₜᵢₐₗ)* / *(A_{Triton-X-100}* - *Aᵢₙᵢₜᵢₐₗ*) × 100%) as the absorbance.

### 4. Characterization of boronsome (dynamic light scattering, ζ potential analysis, morphological examination, three-dimensional model construction)

Dynamic light scattering and zeta potential analysis were performed on Malvern's Zetasizer Nano ZS; and morphological examination of boronsome was performed by TEM at 200 kV (JEM-2100, JEOL, Japan). A drop of boronsome solution was added to the copper mesh covered with ultrathin carbon film, and dried at room temperature for 10 min. Excess solution was removed prior to negative staining with uranyl acetate solution (4% w/v), and then the copper mesh was washed with water. By optimization of density functional theoryBeck, three parameter, Lee-Yang-Parr, and 6-31G* basis sets (DFTB3LYP/6-31G*), the three-dimensional structure of BoPs was predicted using Spartan 14 software. The parameters such as *I_{c}*, carbon arm length (K), *I_{b},* boron arm length (K); *Δl*, the difference in lipid arm length (Å), *d*, lipid arm spacing (Å), and the dihedral angle (°) between the lipid arm were measured and evaluated.

| | BoP-1 | BoP-2 | BoP-3 | BoP-4 | DPPC |
|---|---|---|---|---|---|
| Difference in length (A) | 9.477 | 4.548 | 0.311 | 4.820 | 0.765 |
| Distance between lipid arms (A) | 6.434 | 6.292 | 4.702 | 4.738 | 4.703 |
| Dihedral angle (°) | 52,83 | 32.58 | 1.71 | 9.55 | 3.13 |

### 5. Radiolabeling of Boronsome

⁶⁴CuCl provided by the Cancer Hospital of Peking University was dissolved in 0.01 M hydrochloric acid. NOTA-boronsome were prepared according to the specified composition (BoP-3:DPPC: cholesterol: DSPE-PEG2k-NOTA = 50:10:40:1, by mole percent). To the boronsome solution (100 µL, 0.5 mg/mL) was added NaAc buffer (1 mL, pH = 5.5, 0.2 M), followed by co-incubation with ⁶⁴CuCl₂ (18.5 MBq) for 2 hours at 37°C. Then, the ⁶⁴Cu-NOTA-boronsome was purified using PD-10 size exclusion chromatography with PBS as eluent, and the radiochemical yield was greater than 90%.

Regarding the selection of boron-containing molecules, we selected carborane (C₂B₁₀H₁₂) with high hydrophobicity and low nucleophilicity as the boron-containing molecule binding to the liposome membrane. Carborane had a considerable boron content and could provide a good BNCT efficacy. Therefore, we attached boronated acyl group (carboranated fatty acid) to phospholipid molecule (Lyso-PPC) and utilized Thiol-halo reaction instead of click chemistry to maintain the flexibility of the phospholipids. However, because carborane involves molecule with three-dimensional structure, it is required to consider the effect of steric hindrance on the stability of lipid bilayer formation. Therefore, we screened carboranized fatty acids of different lengths (with the C atom number of 4, 8, 12, and 16 in the carbon chain, respectively, and the C atom number is not limited hereto in practical use). First, we examined the three-dimensional structures of BoPs with different numbers of carbon atoms. Using dipalmitoylphosphatidylcholine DPPC as reference, we compared the lengths of the two lipid arms of carboranated fatty acids with different numbers of carbon atoms. It is found that BoP-3 has the minimum difference in length, which is even less than that of DPPC. It is conducive to the formation of the most stable membrane structure. For other parameters, such as the distance between lipid arms, dihedral angle, etc., it is found that BoP-3 is closest to DPPC. On that basis, and in combination with molecular dynamics simulation, the bilayer system formed with DPPC molecule was simulated for 128 BoP-3s. It is found that the film structure formed by BoP-3 is relatively regular and neat, and has small degree of chain folding and deformation. At the same time, we measured the drug loading capacity of BoP-3 at an experimental level by evaluating the drug loading efficiency in the case of encapsulating sulforhodamine B (SRB). The lipid was hydrated with a 50 mM solution, and then subjected to gel filtration to give the liposome. The highest absorption is found at 550 nm for BoP-3, which is close to the DPPC group. After cultured for 24 hours, the release of SRB was measured in 50% bovine serum at 37°C to assess the stability. It is concluded that the nanostructure formed by BoP-3 exhibits high retention efficacy for SRB. By final screening, BoP-3 (with C atom number of 12 in the carbon chain) is found as the optimal structure for self-assembly.

PET imaging was made using DSPE-PEG2000-NOTA as the chelating agent for positron-emitting nuclide Cu-64. The NOTA-lipid described above was incorporated into the boronsome, and then incubated with an aqueous solution of Cu-64 ions. ⁶⁴Cu-NOTA-Boronsome was purified by gel filtration. Stability was verified by radioactive thin layer chromatography for up to 24 hours. Radiolabeled boronsome (7.4 MBq, 150 µL) was then injected intravenously into 4T1 tumor-bearing mice, and PET-CT imaging was made (FIG. 4) to evaluate the biodistribution and tumor specificity of boronsome in mice. This further verifies that the biodistribution (ratio of tumor/normal site) of this new boronsome in the body is acceptable. In the practice of clinical tumor treatment, it can be used to determine the position where the tumor is located, evaluate the efficacy in real time, and formulate further treatment schemes.

Boron concentration in cells incubated with the boronsome was assessed by ICP-OES. Mouse triple-negative breast cancer 4T1 cells were co-incubated with the boronsome at concentration of 0.1-10 mg/mL for 24 hours. By the experiment, it is found that the intracellular boron concentration gradually increases with the incubation dose of boronsome (FIG. 12). At a dose of 5 mg/mL, the boron concentration in 4T1 cells could reach 182.5 ppm (n=4), which fully meets the clinical requirements of BNCT.

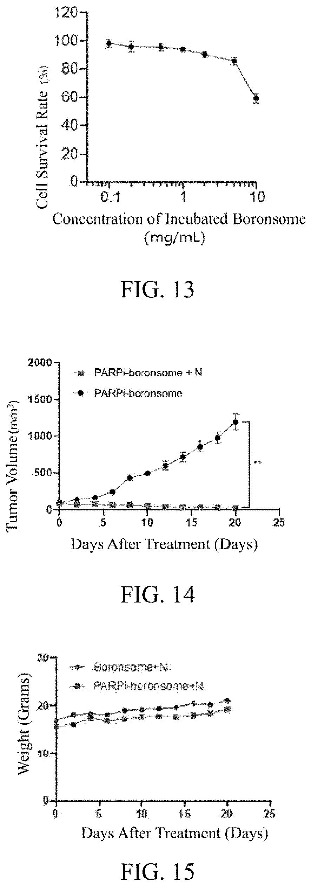

Next, the cytotoxicity of boronsome was evaluated by the CCK-8 assay. As shown in FIG. 13, the cell viability of 4T1 cells incubated with different concentrations of boronsome for 24 hours was measured. By experiments, it is found that cells showed good tolerance to the boronsome when the incubation concentration reached 5 mg/mL.

On the basis of the above studies, a neutron irradiation experiment was performed on 4T1 tumor-bearing mice using the boronsome. PBS (400 µL), the boronsome (500 mg/kg), or the Dox-boronsome (500 mg/kg) were injected via the tail vein to 4T1 tumor-bearing mice. After 12 hours, the mice were anesthetized and fixed in outflow port exit of the slow neutron beam (@IHNI). The mice were arranged to allow the head to orient towards the center, the tumor to expose to full power irradiation in the thermal neutron irradiation range for 30 min, and the liver, spleen and intestine to locate outside the radiation field to avoid unnecessary damage.

The *in vivo* anti-tumor efficacy of the PARPi-boronsome-BNCT was further studied. By the experiment, it is found that the tumor volume of the PARPi@BLNP group (*i.e.*, in which the boronsome was loaded with PARPi but did not undergo a neutron irradiation) significantly increased with the days after treatment; while the average tumor volume of the PARPi@BLNP+N group (*i.e.*, in which the boronsome was loaded with PARPi and underwent a neutron irradiation) was reduced to one-fifth of the original volume (FIG. 14), indicating a better tumor suppression effect than that of the other control groups.

The weight of mice in each group in which the mice were treated with the boronsome and the neutron irradiation or with the boronsome loaded with a PARP inhibitor and neutron irradiation was measured, and the resulting weight curve of mice (FIG. 15) demonstrated that the above treatments did not have a significant toxicity.

In terms of integration of radiotherapy and chemotherapy, the preferred drug for encapsulation was Doxorubicin. It is found that some tumors could be eradicated, which preliminarily verifies the effect of chemotherapy-assisted boronsome-BNCT treatment. Subsequently, we considered that if we could start from the principles of BNCT and selected individual drugs that disrupt the DNA replication process, its efficacy could be further enhanced. Therefore, a poly(ADP-ribose)polymerase (PARP) inhibitor (olaparib) was selected. We verified its interference on the DNA repair system by γ-h2ax staining method. At the same time, it is found by the *in vivo* experiments in 4T1 tumor-bearing mice that the PARPi-boronsome has better efficacy than doxorubicin-boronsome (see FIG. 2).

All references (including literature references, issued patents, published patent applications, and co-pending patent applications) cited throughout this application are incorporated by reference in their entirety. Unless otherwise defined, all technical and scientific terms used herein are to be understood to have the same meaning as commonly understood by one of ordinary skill in the art.

All features disclosed in this specification can be combined in any combination. Each feature disclosed in this specification may be replaced by alternative features serving the same, equivalent or similar purpose. Therefore, unless expressly stated otherwise, each feature disclosed is only one example of a series of equivalent or similar features.

From the above description, those skilled in the art can easily determine the essential characteristics of the present invention, and without departing from the spirit and scope of the invention, can make various changes and modifications to the invention to adapt it to various uses and conditions. Accordingly, other examples are within the scope of the appended claims.

## Claims

1. A carborane-phospholipid conjugate of Formula (I), 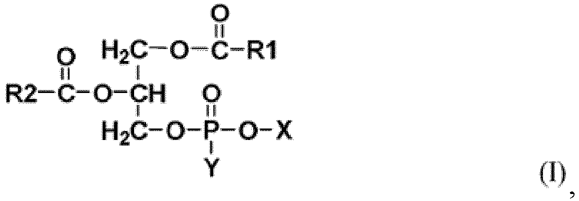 wherein:
one of R1 and R2 is -L-carboranyl, and the other is a linear alkyl or alkenyl group of 10-24 carbon atoms, wherein L is a divalent linking group;
X is -H, -CH₂CH₂NH₃, -CH₂CH₂N⁺(CH₃)₃, -CH₂CH(NH₂)COOH, - CH₂CH(OH)CH₂OH, or 2,3,4,5,6-pentahydroxy-cyclohexan-1-yl;
Y is OH or O⁻.

2. The conjugate according to claim 1, wherein the carboranyl in the -L-carboranyl is ¹⁰B enriched.

3. The conjugate according to any one of claims 1 to 2, wherein X is - CH₂CH₂N⁺(CH₃)₃; and Y is O⁻.

4. The conjugate according to any one of claims 1 to 3, wherein the two lipid arms each comprising R1 or R2 satisfying one, two or three of conditions of:
a difference in length within ± 1 Å;
a distance between lipid arms within 5 Å; and
a dihedral angle within 5 degrees;
preferably, having all of the three.

5. The conjugate according to any one of claims 1 to 4, wherein the carboranyl is 1,2-C₂B₄H₅-, 1,2-C₂B₈H₉-, 1,2-C₂B₁₀H₁₁-, 2,3-C₂B₄H₇-, 7,8-C₂B₉H₁₂-, or 5,6-C₂B₈H₁₁-, preferably 1,2-C₂B₁₀H₁₁-.

6. The conjugate according to any one of claims 1 to 5, wherein L is - (CH₂)ₙ₊ₘ-, -(CH₂)ₙ₊ₘ-S-, -(CH₂)ₙ₊ₘ-O-, -(CH₂)ₙ-CH=CH-(CH₂)ₘ-, -(CH₂)ₙ-CH=CH-(CH₂)ₘ-S-, -(CH₂)ₙ-CH=CH-(CH₂)ₘ-O-, -(CH₂)ₙ-S-(CH₂)ₘ-, -(CH₂)ₙ-S-(CH₂)ₘ₊₁-S-, -(CH₂)ₙ-O-(CH₂)ₘ-, -(CH₂)ₙ-O-(CH₂)ₘ₊₁-O-, -(CH₂)ₙ-N(CH₃)-(CH₂)ₘ-, or -(CH₂)ₙ-N(CH₃)-(CH₂)ₘ₊₁-N(CH₃)-, preferably -(CH₂)ₙ₊ₘ-S-, wherein n is an integer from 1 to 20, and m is an integer from 0 to 20.

7. The conjugate according to claim 6, having a structure of: 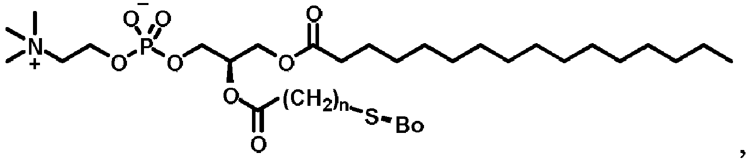 wherein Bo represents a carboranyl, and n is an integer from 1 to 20.

8. The conjugate according to claim 7, wherein
Bo is 1,2-C₂B₁₀H₁₁-, preferably dicarba-closo-dodecylboranyl, more preferably 1,2-dicarba-closo-dodecylboranyl; n is 3, 7, 11 or 15, preferably 11.

9. A liposome composition comprising:
the conjugate according to any one of claims 1-8, wherein the conjugate a is part of a lipid bilayer.

10. The liposome composition according to claim 9, further comprising: cholesterol.

11. The liposome composition according to claim 9 or 10, further comprising a phospholipid, preferably dilauroylphosphatidylcholine (DLPC), dimyristoylphosphatidylcholine (DMPC), distearoylphosphatidylcholine (DSPC), dioleoylphosphatidylcholine (DOPC), dipalmitoylphosphatidylcholine (DPPC), dimyristoylphosphatidylglycerol (DMPG), distearoylphosphatidylglycerol (DSPG), dioleoylphosphatidylglycerol (DOPG), dipalmitoylphosphatidylglycerol (DPPG), dimyristoylphosphatidylserine (DMPS), distearoylphosphatidylserine (DSPS), dioleoylphosphatidylserine (DOPS), dipalmitoylphosphatidylserine (DPPS), dioleoylphosphatidylethanolamine (DOPE), dipalmitoylphosphatidylethanolamine (DPPE), dimyristoylphosphatidylethanolamine (DMPE), distearoylphosphatidylethanolamine (DSPE), or dididaidoylphosphoethanolamine, more preferably DPPC.

12. The liposome composition according to any one of claims 9 to 11, further comprising a PEGylated phospholipid, preferably DLPE-PEG, DPPE-PEG, DMPE-PEG, or DSPE-PEG, more preferably DSPE-PEG.

13. The liposome composition according to any one of claims 9 to 12, further comprising at least one therapeutic agent and/or at least one diagnostic agent.

14. The liposome composition according to claim 13, comprising at least one therapeutic agent, which is an anti-cancer drug.

15. The liposome composition according to claim 14, wherein the anti-cancer drug is selected from the group consisting of monomethyl auristatin E, monomethyl auristatin F, ibrutinib, acalabrutinib, zanubrutinib, doxorubicin, mitomycin-C, mitomycin-A, daunorubicin, aminopterin, actinomycin, bleomycin, 9-aminocamptothecin, N8-acetylspermidine, 1-(2-chloroethyl)-1,2-dimethanesulfonylhydrazide, Yunnanmycin, gemcitabine, cytarabine, dolastatin, dacarbazine, 5-fluorouracil; paclitaxel, docetaxel, gemcitabine, cytarabine, 6-mercaptopurine, vincristine, cisplatin, oxaliplatin, PARP inhibitors.

16. The liposome composition according to claim 15, wherein the anti-cancer drug is selected from PARP inhibitors, preferably olaparib, niraparib, rucaparib, fluzoparib, pamiparib, veliparib, talazoparib, more preferably olaparib.

17. The liposome composition according to claim 13, comprising at least one diagnostic agent, wherein the diagnostic agent is ⁶⁴Cu-NOTA-PEG2000-DSPE.

18. Use of the liposome composition according to any one of claims 9 to 17 in a medicament.

19. Use of the liposome composition according to any one of claims 9 to 17 for delivering at least one therapeutic agent and/or at least one diagnostic agent.
